# FASCICULE DE BREVET EUROPEEN

(11) **EP 3 380 785 B1**
(45) Date de publication et mention de la délivrance du brevet: **07.10.2020**
(21) Numéro de dépôt: 16770051.7
(22) Date de dépôt: 23.08.2016
(51) Int. Cl.: F21V 21/40, F21V 17/16, F21V 17/18, F21W 131/20

(54) **APPAREIL D'ECLAIRAGE AVEC UNE POIGNEE DEMONTABLE**
BELEUCHTUNGSGERÄT MIT ABNEHMBAREM GRIFF
LIGHTING APPLIANCE WITH REMOVABLE HANDLE

(30) Priorité: 26.11.2015 FR 1561411
(43) Date de publication de la demande: 03.10.2018
(73) Titulaire: Maquet SAS, 45160 Ardon (FR)
(72) Inventeur: CHANGEUX, Patrice, 45160 Ardon (FR); LOISEAU, André, 45160 Ardon (FR)
(74) Mandataire: Prugneau, Philippe
(86) Numéro de dépôt international: PCT/FR2016/052106
(87) Numéro de publication internationale: WO 2017/089661

(56) Documents cités:
- EP-A1- 1 568 934
- CN-A- 101 769 482
- JP-A- H04 312 457
- US-A- 3 075 071
- US-A- 4 316 237
- US-A- 5 493 757
- US-A- 5 604 955
- US-A1- 2003 014 834
- US-A1- 2011 317 411

## Description

### Domaine technique

L'invention concerne de façon générale un appareil d'éclairage qui comprend un module de lumière avec une embase destinée à recevoir une poignée pourvue d'un manche de préhension, la poignée étant agencée pour s'encliqueter de façon démontable par insertion axiale dans une bague agencée sur l'embase.

### Technique antérieure

De manière connue, un appareil d'éclairage utilisé dans un bloc opératoire pour l'éclairage d'un champ opératoire comprend un module de lumière accroché à un bras de suspension de sorte à être positionnable au dessus du champ opératoire. La position du module de lumière est modifiable par l'équipe médicale du bloc opératoire. Pour manœuvrer le module de lumière afin de modifier sa position, celui-ci est pourvu d'une poignée permettant de le déplacer et de l'orienter dans l'espace au dessus du champ opératoire.

Il est actuellement de pratique courante de fournir des poignées stériles ou un couvercle stérile de poignées qui seront touchés par le personnel de l'équipe médicale lors de la manipulation du module d'éclairage pendant une intervention.

On connait des documents US4844252 et US6370735 des poignées stériles connectables de façon amovibles à un module de lumière d'un éclairage opératoire. Ces poignées présentent une partie supérieure filetée pour être vissée dans une douille agencée sur la structure du module d'éclairage. Le document US 4 316 237 A divulgue également une poignée amovible.

Dans un bloc opératoire, afin de maintenir une stérilité des lieux, il est nécessaire de limiter les manipulations, les surfaces touchées, en facilitant les connections entre différents équipements médicaux. Or, avec les poignées connues, le montage/démontage de la poignée sur le module d'éclairage nécessite qu'un couple important soit appliqué lors des étapes de vissage, dévissage. De plus au cours de ces opérations de vissage, dévissage, en plus de tenir la poignée par un manche, le personnel médical doit aussi toucher le module d'éclairage pour le maintenir en place puisqu'il est agencé sur un bras de suspension qui bouge.

### Exposé de l'invention

Le but de l'invention est de remédier à ces inconvénients en proposant un appareil d'éclairage permettant de monter et démonter facilement et rapidement une poignée stérile. L'invention a pour objet un appareil d'éclairage selon la revendication 1 ou la revendication 2.

Plus particulièrement, l'invention a pour objet un appareil d'éclairage comprenant un module de lumière avec une embase destinée à recevoir une poignée pourvue d'un manche de préhension, la poignée étant agencée pour s'encliqueter de façon démontable par insertion axiale dans une bague agencée sur ladite embase, caractérisé en ce que la poignée peut être retirée axialement de l'embase par rotation de la bague par rapport à l'embase.

L'appareil d'éclairage médical selon l'invention peut encore présenter les particularités suivantes :
--un verrou peut être logé dans la bague pour bloquer et débloquer la poignée dans l'embase ;
-le verrou peut comprendre au moins une came rétractable ;
-le verrou peut comprendre deux cames montées sur ressorts de sorte que les ressorts pressent les cames pour bloquer la poignée dans l'embase et que lors de la rotation de la bague, les ressorts peuvent être compressés par les cames poussées par des ergots agencés sur la surface interne de la bague ;
-le verrou peut comprendre au moins un pion rétractable s'étendant radialement ;
-le verrou peut comprendre trois pions montés sur ressorts de sorte que les pions peuvent être en saillie dans la bague pour bloquer la poignée dans l'embase et que lors de la rotation de la bague, les pions peuvent être en position rétractée dans un logement agencé sur la surface interne de la bague ;
-la poignée peut comprendre une tête de fixation avec un plot de positionnement dans la bague de forme bombée, le plot ayant une rainure annulaire agencée pour pouvoir coopérer avec le verrou ;
-la poignée peut être stérile ;
-un appareil électrique peut être logé dans la poignée et des moyens d'alimentation électrique et de contrôle/commande de l'appareil électrique peuvent être agencés sur la poignée et l'embase.

Avec cet agencement selon l'invention, on obtient un appareil d'éclairage médical sur lequel il est possible de monter rapidement par encliquetage une poignée stérile à une embase de l'appareil d'éclairage et de verrouiller/déverrouiller facilement la poignée dans l'embase grâce à une bague rotative de l'embase de l'appareil d'éclairage que le personnel médical peut aisément faire tourner par rapport à l'embase.

Selon l'invention, l'ajout d'un verrou dans la bague permet de coopérer avec la poignée pour lorsque la bague est en position de verrouillage pouvoir retenir la poignée dans l'embase, et lorsque la bague est en position de déverrouillage pouvoir autoriser le retrait de la poignée. Le verrou comprend des moyens de retenue rétractables, à came ou à pion, montés sur ressort de sorte à être en saillie ou en position rétractée dans l'orifice de la bague en fonction de la position de la bague rotative.

Avec l'appareil d'éclairage selon l'invention, les manipulations de l'appareil et les surfaces touchées par personnel médical pour connecter une poignée sont respectivement facilitées et limitées.

### Présentation sommaire des dessins

La présente invention sera mieux comprise et d'autres avantages apparaîtront à la lecture de la description qui suit et des dessins annexés dans lesquels :
- la figure 1 est une représentation schématique d'un appareil d'éclairage médical selon l'invention qui est manipulé par un opérateur par l'intermédiaire d'une poignée ;
- la figure 2 est une vue en perspective d'une poignée pouvant se monter et se démonter à une embase d'un appareil d'éclairage selon l'invention ;
- la figure 3A est une vue en perspective d'une poignée montée à un appareil d'éclairage selon l'invention ;
- la figure 3B est une autre vue en perspective d'une poignée montée à un appareil d'éclairage selon l'invention ;
- la figure 4 est une vue en perspective éclatée d'une embase d'un appareil d'éclairage médical et d'une poignée selon un premier mode de réalisation de l'invention ;
- la figure 5A est une vue de profil d'une poignée montée dans une embase d'un appareil d'éclairage médical selon un premier mode de réalisation de l'invention ;
- la figure 5B est une vue en coupe longitudinale de la figure 5A ;
- les figures 6A et 6B sont des vues en coupe transversale illustrant respectivement les positions de déverrouillage et de verrouillage de l'embase pour une poignée d'un appareil d'éclairage médical selon un premier mode de réalisation de l'invention ;
- la figure 7 est une vue en perspective éclatée d'une embase d'un appareil d'éclairage médical et d'une poignée selon un second mode de réalisation de l'invention ;
- la figure 8 est une vue de profil en coupe longitudinale d'une partie d'une poignée montée dans une embase d'un appareil d'éclairage médical selon un second mode de réalisation de l'invention ;
- les figures 9A et 9B sont des vues en coupe transversale illustrant respectivement les positions de déverrouillage et de verrouillage de l'embase pour une poignée d'un appareil d'éclairage médical selon un second mode de réalisation de l'invention ;
- les figures 10A, 10B et 10C illustrent une poignée démontable adaptée pour loger un appareil électrique, avec deux vues en perspectives d'une poignée pouvant se monter et se démonter à une embase et une vue en perspective éclatée d'une embase d'un appareil d'éclairage médical et d'une poignée selon un troisième mode de réalisation de l'invention.

### Description d'un mode de réalisation

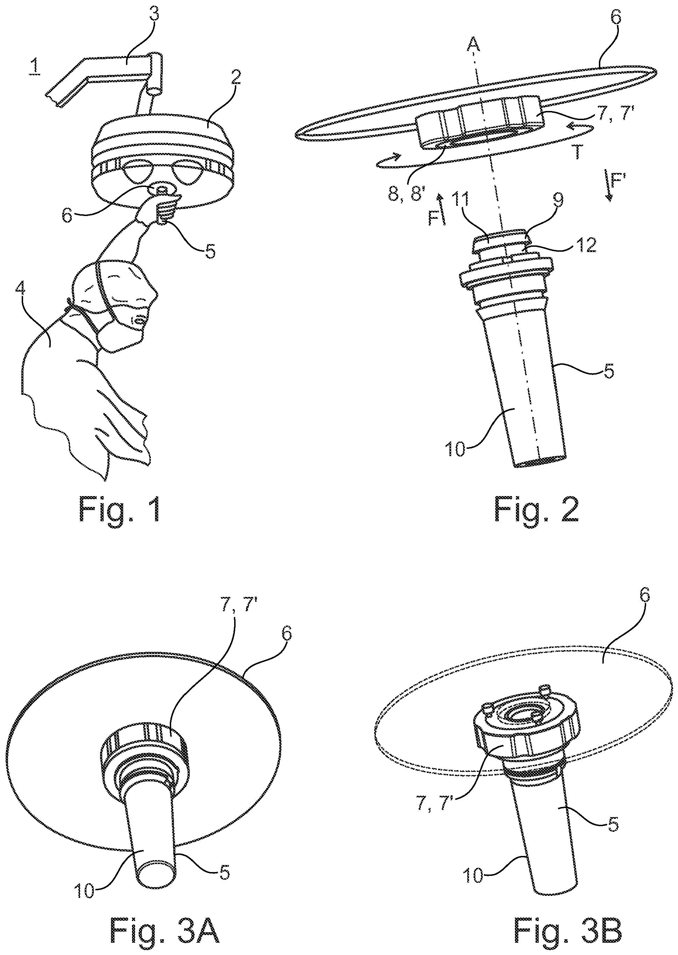

La figure 1 illustre une partie d'un appareil d'éclairage 1 médical utilisé dans un bloc opératoire, avec un module de lumière 2 accroché à un bras 3 de suspension articulé, de sorte à pouvoir positionner de façon ajustable le module de lumière 2 au dessus d'un champ opératoire (non représenté) et de l'éclairer de façon approprié. Ici, un membre du personnel médical 4 tel qu'un chirurgien manœuvre le module de lumière 2, à l'aide d'une poignée 5, pour le déplacer et l'orienter au dessus du champ opératoire.

La poignée 5 est connectée de façon démontable au module de lumière 2 par l'intermédiaire d'une embase 6, agencée ici de façon centrée sur une face inférieure du module de lumière 2.

L'embase 6, représentée ici est sous forme d'un disque plat. Une des faces du disque est fixée par tous moyens connus au module de lumière 2. Comme visible sur la figure 2, sur l'autre face du disque est agencé un système de fixation de la poignée 5 dans l'embase 6. Le système de fixation est composé d'une bague 7, 7' agencée sur l'embase 6 de sorte qu'elle puisse tourner autour un axe A perpendiculaire à la surface du disque de l'embase 6, la rotation pouvant se produire dans un sens ou dans l'autre selon la double flèche T.

Dans l'orifice formé par la bague 7, 7' est logé un verrou 8, 8', emboitable axialement dans la bague 7, 7' et qui sera décrit plus loin. Le verrou 8, 8' est destiné à maintenir la poignée 5 dans l'embase 6 lorsque la poignée 5 est insérée axialement selon la flèche F dans la bague 5 et de retirer axialement la poignée 5 de l'embase 6 selon la flèche F', par rotation de la bague 7, 7' .

La poignée 5 comprend une tête 9 de fixation portée par l'une des extrémités d'un manche de préhension 10.

Les figures 3A et 3B sont des illustrations de la poignée 5 encliquetée dans l'embase 6, la tête 9 de fixation de la poignée 5 étant en prise dans la bague 7, 7' et est maintenue dans la bague 7, 7' par l'intermédiaire du verrou 8, 8'. La poignée 5 reste accessible par le manche de préhension 10.

Comme visible sur les figures 2, 4 et 7, la tête 9 de fixation comprend une base sous forme d'un disque avec un plot 11 de positionnement s'étendant axialement. Le plot 11 de positionnement présente ici une forme bombée pour faciliter l'insertion de la tête 9 de fixation dans l'orifice de la bague 7, 7' et comporte une rainure 12 annulaire qui coopère avec le verrou 8, 8' pour maintenir la poignée 5 dans l'orifice de la bague 7, 7'.

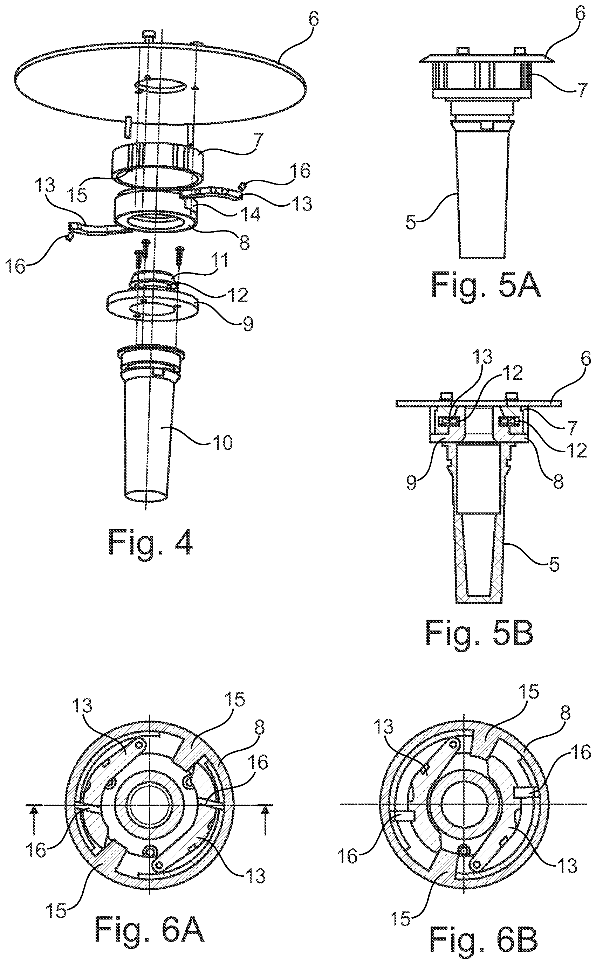

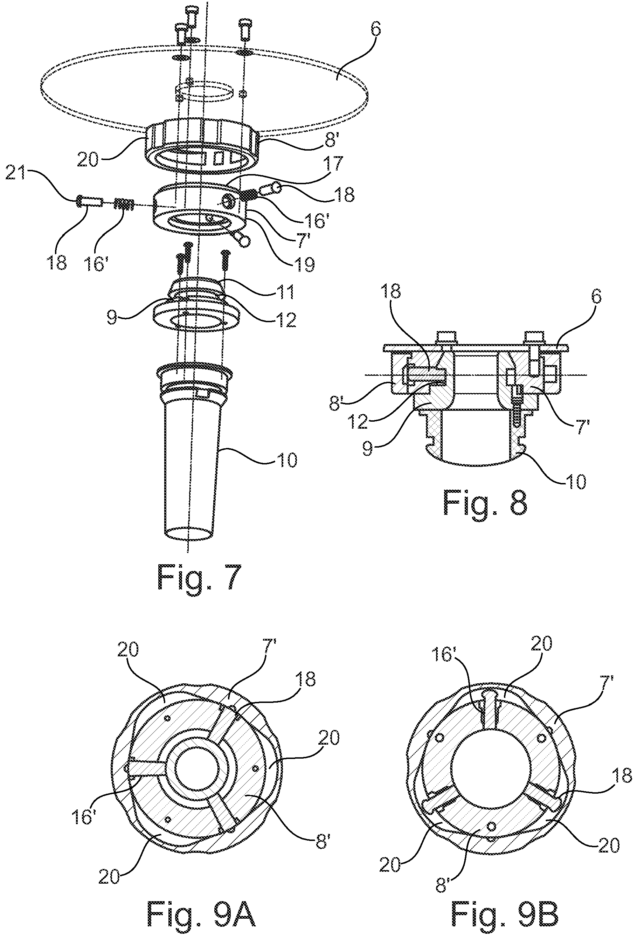

Les figures 4 à 6 illustrent un premier mode de réalisation d'encliquetage de façon démontable de la poignée 5 dans l'embase 6, les figures 7 à 9 illustrent un second mode de réalisation d'encliquetage de façon démontable de la poignée 5 dans l'embase 6. Les éléments communs aux deux modes de réalisation portent les mêmes références.

La figure 4 illustre de façon éclatée un premier mode de réalisation d'encliquetage de façon démontable de la poignée 5 dans l'embase 6. Dans ce mode de réalisation, le verrou 8 se présente sous forme d'un anneau, de diamètre extérieur légèrement inférieur au diamètre interne de la bague 7. L'anneau du verrou 8 présente deux cames 13 en forme de spirale agencées de façon symétrique, montées de façon pivotante à l'une de leur extrémité sur la circonférence de l'anneau et s'étendant dans des ouvertures 14 le long de la circonférence de l'anneau.

La bague 7 présente ici sur sa surface interne deux ergots 15 en saillie radialement, agencés de façon symétrique (visibles sur les figures 6A et 6B).

Les figures 5A et 5B illustrent la poignée 5 montée dans l'embase 6 de l'appareil d'éclairage selon le premier mode de réalisation avec la bague 7 en position de verrouillage.

Les figures 6A et 6B illustrent le principe de fonctionnement du verrou 8 en fonction de la position de la bague 7, c'est-à-dire en position de verrouillage (figure 6B) ou en position de déverrouillage (figure 6A).

Lorsque le verrou 8 est logé dans la bague 7, un ressort 16 est présent dans l'espace entre chaque extrémité libre des cames 13 et la surface interne de la bague 7 de sorte qu'en position de verrouillage (figure 6B) de la bague 7 les cames 13 sont en saillie dans l'orifice du verrou 8 à travers les ouvertures 14 du verrou. Dans la position de déverrouillage de la bague 7 (figure 6A), les ergots 15 de la bague 8 appuient sur les extrémités libres des cames 13 engendrant un retrait des cames 13 dans les ouvertures 14 du verrou 8 et une compression des ressorts 16 entre les extrémités libres des cames 13 et la surface interne de la bague 7.

La figure 7 illustre de façon éclatée un second mode de réalisation d'encliquetage de façon démontable de la poignée 5 dans l'embase 6 de l'appareil d'éclairage 1. Dans ce mode de réalisation, le verrou 8' se présente aussi sous forme d'un anneau, de diamètre extérieur légèrement inférieur au diamètre interne de la bague 7'.

Le verrou 8' présente ici trois orifices 17 disposés radialement et symétriquement dans la paroi du verrou 8' par lesquels des pions 18, de longueur supérieure à l'épaisseur du verrou 8(, sont montés sur ressorts 16' et sont disposés de façon traversant.

Les pions 18 présentent une tête de diamètre plus large que le diamètre de l'orifice 17.

Chaque orifice 17 présente un épaulement 19 de sorte que le ressort 16' ne traverse pas l'orifice 17 du coté de la face interne du verrou 8'.

La bague 7' présente ici sur sa surface interne trois évidements 20 disposés symétriquement (visibles sur les figures 9A et 9B).

Les figures 8 et 9A illustrent le verrou 8' logé dans la bague 7' en position de verrouillage selon le second mode de réalisation. Dans cette position de verrouillage de la bague 7', les têtes 21 des pions 18 sont poussées dans les orifices 17 par la surface interne « pleine » (c'est-à-dire en dehors des évidements 20) de la bague 7'. Les pions 18 sont en saillie dans l'orifice du verrou 8' et les ressorts 16' sont compressés.

La figure 9B illustre le verrou 8' et la bague 7' en position de déverrouillage. Dans cette position de déverrouillage les évidements 20 de la bague 7' se trouvent face aux orifices 17 traversant le verrou 8'. Par effet ressort, les pions 18 reculent dans cet espace de sorte qu'ils ne soient plus saillant du coté de l'orifice du verrou 8'.

Dans le premier mode de réalisation décrit ci-dessus et selon l'invention, lorsque le personnel médical 4 veut installer une poignée 5 stérile sur l'appareil d'éclairage 1, il tient la poignée 5 par son manche de préhension 10 et insère axialement la tête 9 de fixation dans l'orifice du verrou 8.

Si la bague 7 est en position de déverrouillage alors les cames 13 sont en retrait dans le verrou 8. En tournant la bague 7 en position de verrouillage les cames 13 montées sur ressorts 16 vont venir se loger dans la rainure 12 annulaire de la tête 9 de fixation de la poignée 5 de sorte à bloquer la poignée 5 dans l'embase 6.

Il est à noter que dans ce mode de réalisation, si la bague 7 est en position de verrouillage lors de l'insertion axiale de la poignée 5, la forme bombée de la tête 9 de fixation peut pousser les cames 13 montées sur ressort 16 de sorte qu'elles s'écartent et se rétractent dans les ouvertures 14 du verrou 8 pour céder le passage à la tête 9 de fixation jusqu'au niveau de la rainure 12 annulaire dans laquelle, à ce moment là, les cames 13 montées sur ressort 16 vont automatiquement venir s'y loger pour ainsi bloquer la poignée 5 dans l'embase 6.

Pour démonter la poignée 5 de l'appareil d'éclairage 1 afin de la changer, le personnel médical 4 attrape d'une main la poignée 5 par le manche de préhension 10 et de l'autre main tourne la bague 7 en position de déverrouillage. Dans cette position de la bague 7, les ergots 15 de la bague 7 poussent les extrémités libres des cames 13 de sorte à compresser les ressorts 16 pour écarter les cames 13 de l'orifice du verrou 8 et les désengager de la rainure 12 annulaire de la tête 9 de fixation de la poignée 5. Ainsi, le personnel médical 4 peut tirer axialement la poignée 5 pour la retirer de l'embase 6.

Dans le second mode de réalisation décrit ci-dessus et selon l'invention, lorsque le personnel médical 4 veut installer une poignée 5 stérile sur un module de lumière 2 de l'appareil d'éclairage 1, il tient la poignée 5 par son manche de préhension 10 et insère axialement la tête 9 de fixation dans l'embase 6 lorsque la bague 7' est en position de déverrouillage. Dans cette position, les pions 18 ne sont pas en saillie dans l'orifice du verrou 8' et laisse le passage à la tête 9 de fixation. Puis en tournant la bague 7' en position de verrouillage les pions 18 montés sur ressort 16' vont venir se loger dans la rainure 12 annulaire de la tête 9 de fixation de la poignée 5 de sorte à bloquer la poignée 5 dans l'embase 6.

Pour démonter la poignée 5 de l'appareil d'éclairage 1 afin de la changer, le personnel médical 4 attrape d'une main la poignée 5 par le manche de préhension 10 et de l'autre main tourne la bague 7' en position de déverrouillage. Dans cette position de la bague 7', les pions 18 montés sur ressort 16' se trouvent face aux évidements 20 dans lesquels ils y reculent. Les pions 18 se désengagent alors de la rainure 12 annulaire de la tête 9 de fixation de la poignée 5. Ainsi, le personnel médical 4 peut tirer axialement la poignée 5 pour la retirer de l'embase 6.

Dans le milieu médical, maintenir de bonnes conditions de stérilité environnant un champ opératoire est une priorité. L'appareil d'éclairage 1 selon l'invention permet au personnel médical 4 d'encliqueter de façon démontable la poignée 5 en touchant uniquement le manche de préhension 10 et la bague 7, 7'. Ainsi, les surfaces touchées sont limitées pour fixer une poignée 5 stérile préalablement à une opération et la démonter en fin d'opération. De plus, la fixation et le démontage de la poignée 5 sont faciles et rapides. Selon le premier mode de réalisation il est possible de fixer la poignée 5 en poussant la poignée 5 dans l'embase 6, ou par le simple geste de tourner la bague 7 pour verrouiller la poignée 5. Selon le second mode de réalisation il est possible de fixer la poignée 5 par le simple geste de tourner la bague 7' pour verrouiller et déverrouiller la poignée 5 dans l'embase 6.

Selon les premier et second modes de réalisation, pour retirer la poignée 5 de l'embase 6, il suffit de tourner la bague 7, 7' en position de déverrouillage et de tirer axialement la poignée 5.

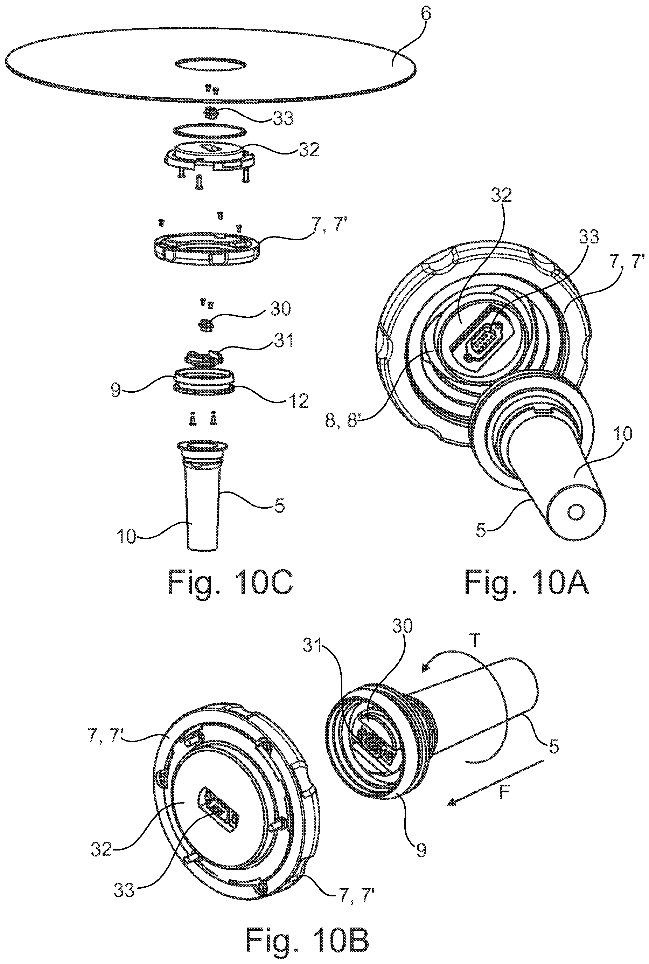

Selon un troisième mode de réalisation de l'invention représenté sur les figures 10A à 10C, la poignée 5 peut être creuse pour d'y loger par exemple une caméra pour filmer le champ opératoire, une lampe, un appareil multimédia ou y loger tout autre appareil électrique (non représenté). Pour pouvoir alimenter électriquement et contrôler ou commander l'appareil électrique logé dans la poignée 5 creuse, des moyens d'alimentation électrique et de contrôle/commande de l'appareil électrique sont agencés sur la poignée et l'embase. Ici le plot 11 de positionnement présente un connecteur 30 rotatif agencé sur un premier support de connecteur 31.

Dans ce mode de réalisation particulier, un second support de connecteur 32 pour un connecteur 33 fixe est monté sur l'embase 6 de sorte que la connexion entre le connecteur 31 rotatif et le connecteur 33 fixe se produise à travers le système de fixation selon l'invention.

La poignée 5 s'encliquète de façon démontable comme décrit précédemment dans la bague 7, 7' dans laquelle est logé avec le verrou 8, 8'.

## Revendications

1. Appareil d'éclairage (1) comprenant un module de lumière (2) avec une embase (6) destinée à recevoir une poignée (5) pourvue d'un manche de préhension (10), ladite poignée (5) étant agencée pour s'encliqueter de façon démontable par insertion axiale dans une bague (7) agencée sur ladite embase (6), **caractérisé en ce que** ladite poignée (5) est retirée axialement de ladite embase (6) par rotation de ladite bague (7) par rapport à ladite embase (6).

2. Appareil d'éclairage (1) comprenant un module de lumière (2) avec une embase (6) destinée à recevoir une poignée (5) pourvue d'un manche de préhension (10), ladite poignée (5) étant agencée pour s'insérer axialement de façon démontable dans une bague (7') agencée sur ladite embase (6), dans lequel un verrou (8') est logé dans ladite bague (7') pour bloquer et débloquer ladite poignée (5) dans ladite embase (6), dans lequel ledit verrou (8') comprend au moins un pion (18) rétractable s'étendant radialement dans ladite bague (7'), dans lequel ladite poignée (5) est bloquée dans ladite embase (6) par ledit au moins un pion (18) et charactérisé en ce que ladite poignée (5) est retirée axialement de ladite embase (6) par rotation de ladite bague (7') par rapport à ladite embase (6).

3. Appareil d'éclairage (1) selon la revendication 1, **caractérisé en ce qu'**un verrou (8) est logé dans ladite bague (7) pour bloquer et débloquer ladite poignée (5) dans ladite embase (6).

4. Appareil d'éclairage (1) selon les revendications 1 et 3, **caractérisé en ce que** ledit verrou (8) comprend au moins une came (13) rétractable.

5. Appareil d'éclairage (1) selon la revendication 4, **caractérisé en ce que** ledit verrou (8) comprend deux cames (13) montées sur ressorts (16) de sorte que lesdits ressorts (16) pressent lesdites cames (13) pour bloquer ladite poignée (5) dans ladite embase (6) et que lors de ladite rotation de ladite bague (8), lesdits ressorts (16) sont compressés par lesdites cames (13) poussées par des ergots (15) agencés sur la surface interne de ladite bague (7).

6. Appareil d'éclairage (1) selon la revendication 2, **caractérisé en ce que** ledit verrou (8') comprend trois pions (18) montés sur ressorts (16') de sorte que lesdits pions (18) sont en saillie dans ladite bague (7') pour ledit blocage de ladite poignée (5) dans ladite embase (6) et que lors de ladite rotation de ladite bague (8'), lesdits pions (18) sont en position rétractée dans un logement (20) agencé sur la surface interne de ladite bague (8').

7. Appareil d'éclairage (1) selon l'une des revendications 2 à 6, **caractérisé en ce que** ladite poignée (5) comprend une tête (9) de fixation avec un plot (11) de positionnement dans ladite bague (7, 7') de forme bombée, ledit plot (11) ayant une rainure (12) annulaire agencée pour coopérer avec ledit verrou (8, 8').

8. Appareil d'éclairage (1) selon l'une des revendications précédentes, **caractérisé en ce que** ladite poignée (5) est stérile.

9. Appareil d'éclairage (1) selon l'une des revendications précédentes, **caractérisé en ce que** ladite poignée (5) est creuse.

10. Appareil d'éclairage (1) selon la revendication 10, **caractérisé en ce qu'**un appareil électrique est logé dans ladite poignée (5) creuse, et **en ce que** des moyens d'alimentation électrique et de contrôle/commande dudit appareil électrique sont agencés sur ladite poignée (5) et ladite embase (6).

11. Appareil d'éclairage (1) selon la revendication 9 ou 10, **caractérisé en ce qu'**une caméra est logée dans ladite poignée (5) creuse.

12. Appareil d'éclairage (1) selon l'une quelconque des revendications précédentes, **caractérisé en ce que** ledit module de lumière (2) est accroché à un bras de suspension articulé (3) de sorte à positionner de façon ajustable ledit module de lumière (2).

13. Appareil d'éclairage (1) selon l'une quelconque des revendications précédentes, **caractérisé en ce que** ledit appareil d'éclairage (1) est un appareil d'éclairage médical conçu pour éclairer un champ opératoire.

## Patentansprüche

1. Beleuchtungsgerät (1), umfassend ein Lichtmodul (2) mit einem Sockel (6), der dazu bestimmt ist, einen Griff (5) mit einem Greifstiel (10) aufzunehmen, wobei der Griff (5) ausgebildet ist, um sich auf demontierbare Weise durch axiales Einfügen in einen an dem Sockel (6) angeordneten Ring (7) einzuklipsen,
**dadurch gekennzeichnet,**
**dass** der Griff (5) axial von dem Sockel (6) durch Rotation des Rings (7) in Bezug auf den Sockel (6) herausgezogen wird.

2. Beleuchtungsgerät (1), umfassend ein Lichtmodul (2) mit einem Sockel (6), der dazu bestimmt ist, einen Griff (5) mit einem Greifstiel (10) aufzunehmen, wobei der Griff (5) ausgebildet ist, um sich auf demontierbare Weise axial in einen an dem Sockel (6) angeordneten Ring (7') einzufügen, bei dem ein Riegel (8') in dem Ring (7') angeordnet ist, um den Griff (5) in dem Sockel (6) zu verriegeln und entriegeln, bei dem der Riegel (8') wenigstens einen zurückziehbaren Stift (18) umfasst, der sich radial in dem Ring (7') erstreckt, bei dem der Griff (5) in dem Sockel (6) durch den wenigstens einen Stift (18) verriegelt ist, und
**dadurch gekennzeichnet,**
**dass** der Griff (5) axial von dem Sockel (6) durch Rotation des Rings (7') in Bezug auf den Sockel (6) herausgezogen wird.

3. Beleuchtungsgerät (1) nach Anspruch 1, **dadurch gekennzeichnet, dass** in dem Ring (7) ein Riegel (8) zum Verriegeln und Entriegeln des Griffs (5) in dem Sockel (6) angeordnet ist.

4. Beleuchtungsgerät (1) nach den Ansprüchen 1 und 3, **dadurch gekennzeichnet, dass** der Riegel (8) wenigstens eine zurückziehbare Nocke (13) umfasst.

5. Beleuchtungsgerät (1) nach Anspruch 4, **dadurch gekennzeichnet, dass** der Riegel (8) zwei Nocken (13) umfasst, die auf Federn (16) derart montiert sind, dass die Federn (16) die Nocken (13) spannen, um den Griff (5) im dem Sockel (6) zu blockieren, und dass bei der Rotation des Rings (8) die Federn (16) durch die Nocken (13), die von auf der Innenfläche des Rings (7) angeordnete Vorsprüngen (15) verschoben werden, gespannt werden.

6. Beleuchtungsgerät (1) nach Anspruch 2, **dadurch gekennzeichnet, dass** der Riegel (8') drei auf Federn (16') montierte Stifte (18) derart umfasst, dass die Stifte (18) in den Ring (7') für das Blockieren des Griffs (5) in dem Sockel (6) vorstehen, und dass bei der Rotation des Rings (8') die Stifte (18) in zurückgezogener Position in einer Aufnahme (20), die auf der Innenfläche des Rings (8') ausgebildet ist, zurückgezogen sind.

7. Beleuchtungsgerät (1) nach einem der Ansprüche 2 bis 6, **dadurch gekennzeichnet, dass** der Griff (5) einen Fixierkopf (9) mit einem Positionierkegel (11) in dem Ring (7, 7') mit bombierter Gestalt umfasst, wobei der Kegel (11) eine zum Zusammenwirken mit dem Riegel (8, 8') ausgebildete ringförmige Nut (12) aufweist.

8. Beleuchtungsgerät (1) nach einem der vorhergehenden Ansprüche,
**dadurch gekennzeichnet, dass** der Griff (5) steril ist.

9. Beleuchtungsgerät (1) nach einem der vorhergehenden Ansprüche, **dadurch gekennzeichnet, dass** der Griff (5) hohl ist.

10. Beleuchtungsgerät (1) nach Anspruch 10, **dadurch gekennzeichnet, dass** ein elektrischer Apparat in dem hohlen Griff (5) aufgenommen ist, und dass elektrische Versorgungs- sowie Regel-/Steuermittel des elektrischen Apparats an dem Griff (5) und dem Sockel (6) angeordnet sind.

11. Beleuchtungsgerät (1) nach Anspruch 9 oder 10, **dadurch gekennzeichnet, dass** eine Kamera in dem hohlen Griff (5) aufgenommen ist.

12. Beleuchtungsgerät (1) nach einem der vorhergehenden Ansprüche, **dadurch gekennzeichnet, dass** das Lichtmodul (2) an einem gelenkigen Aufhängarm (3) derart angeschlossen ist, dass das Lichtmodul (2) auf justierbare Weise positionierbar ist.

13. Beleuchtungsgerät (1) nach einem der vorhergehenden Ansprüche, **dadurch gekennzeichnet, dass** das Beleuchtungsgerät (1) ein medizinisches Beleuchtungsgerät ist, das zum Beleuchten eines Operationsfelds ausgebildet ist.

## Claims

1. A lighting appliance (1) comprising a light module (2) having a base (6) designed to receive a handle (5) provided with a grip stick (10), said handle (5) being arranged to snap-fastening in removable manner by axial insertion into a ring (7) arranged on said base (6), said lighting appliance being **characterized in that** said handle (5) is withdrawn axially from said base (6) by turning said ring (7) relative to said base (6).

2. A lighting appliance (1) comprising a light module (2) having a base (6) designed to receive a handle (5) provided with a grip stick (10), said handle (5) being arranged such that it can be inserted axially in removable manner into a ring (7') arranged on said base (6), wherein a latch (8') is received in said ring (7') for the purpose of locking and unlocking said handle (5) in said base (6), wherein said latch (8') comprises at least one retractable stud (18) extending radially in said ring (7'), wherein said handle (5) is locked in said base (6) by said at least one stud (18) and said lighting appliance being **characterized in that** said handle (5) is withdrawn axially from said base (6) by turning said ring (7') relative to said base (6).

3. A lighting appliance (1) according to claim 1, **characterized in that** a latch (8) is received in said ring (7) for the purpose of locking and unlocking said handle (5) in said base (6).

4. A lighting appliance (1) according to claims 1 and 3, **characterized in that** said latch (8) is provided with at least one retractable cam (13).

5. A lighting appliance (1) according to claim 4, **characterized in that** said latch (8) comprises two cams (13) mounted on springs (16) so that said springs (16) press said cams (13) so as to lock said handle (5) in said base (6), and so that, when said ring (8) is turned, said springs (16) are compressed by said cams (13) as pushed by lugs (15) arranged on the inside surface of said ring (7).

6. A lighting appliance (1) according to claim 2, **characterized in that** said latch (8') is provided with three studs (18) mounted on springs (16') so that said studs (18) project into said ring (7') to lock said handle (5) in said base (6) and so that, when said ring (8') is turned, said studs (18) are in a retracted position in which they are retracted into a recess (20) arranged in the inside surface of said ring (8').

7. A lighting appliance (1) according to any preceding claim 2 to 6, **characterized in that** said handle (5) is provided with a fastening head (9) having a positioning protuberance (11) of convex shape for positioning in said ring (7, 7'), said protuberance (11) having an annular groove (12) arranged to co-operate with said latch (8, 8').

8. A lighting appliance (1) according to any preceding claim, **characterized in that** said handle (5) is sterile.

9. A lighting appliance (1) according to any preceding claim, **characterized in that** said handle (5) is hollow.

10. A lighting appliance (1) according to claim 9, **characterized in that** an electrical appliance is received in said hollow handle (5), and **in that** means for electrically powering and monitoring/controlling said electrical appliance are arranged on said handle (5) and on said base (6).

11. A lighting appliance (1) according to claim 9 or 10, **characterized in that** a camera is received inside said hollow handle (5).

12. A lighting appliance (1) according to any preceding claim, **characterized in that** said light module (2) is hooked to an articulated arm (3) so as to position in an adjustable manner said light module (2).

13. A lighting appliance (1) according to any preceding claim, **characterized in that** said lighting appliance (1) is a medical lighting appliance designed to illuminate an operating field.
